# EUROPÄISCHE PATENTANMELDUNG

(11) **EP 1 854 872 A1**
(43) Veröffentlichungstag der Anmeldung: **14.11.2007**
(21) Anmeldenummer: 07007790.4
(22) Anmeldetag: 17.04.2007
(51) Int. Cl.: C12M 1/00, C12M 1/26

(54) **Reaktions- und/oder Anzuchtcontainer und Containersystem für Mikroorganismen und Zellkulturen**

(30) Priorität: 12.05.2006 CH 7642006
(71) Anmelder: Röll, Marcel, 8124 Maur (CH)
(72) Erfinder: Röll, Marcel, 8124 Maur (CH)
(74) Vertreter: Kleinschmidt, Michael

(57) **Zusammenfassung**

Um bei einem Kunststoffcontainer unerwünschte Strömungen beim Befüllen zu vermeiden und um weiterhin zu vermeiden dass sich bei solchen Kunststoffcontainern Sedimente ablagern oder dass Totvolumina ausgebildet werden, wird ein Kunststoffcontainer (10) vorgeschlagen, der eine zylindrische oder nahezu zylindrische Form aufweist. Er umfasst einen integrierten Containerboden (12) und einen Deckel (14). Dabei ist der Containerboden (12) so geformt, dass er angeschrägt ausläuft. Anschrägungen von ca. 30° bis 40° haben sich als vorteilhaft erwiesen.

## Beschreibung

### Technisches Gebiet

Die Erfindung betrifft ein Containersystem für biotechnische Reaktionen mit Durchmischung und/oder die Anzucht von Mikroorganismen und Zellkulturen.

### Stand der Technik

Herkömmliche Kunststoffcontainer mit einem Volumen von ca. 50 bis 2000 Liter für biotechnische Reaktionen und/oder die Anzucht von Mikroorganismen und Zellkulturen sind üblicherweise als beutelförmige Kunststoffbehälter geformt. Bei solchen Kunststoffcontainern hat es sich aber als nachteilig herausgestellt, dass beim Befüllen und Durchmischen des Kunststoffcontainers unerwünschte Strömungen auftreten. Weiterhin nachteilig erscheint bei den herkömmlichen Kunststoffcontainern, dass sich leicht Sedimente ablagern und auch Totvolumina ausgebildet werden.

### Darstellung der Erfindung

Aufgabe der Erfindung ist es, einen Kunststoffcontainer für biotechnische Reaktionen und/oder die Anzucht von Mikroorganismen und Zellkulturen so auszubilden, dass die oben genannten Nachteile vermieden werden. Weiterhin ist es die Aufgabe der Erfindung, ein Unterstützungssystem für solche Kunststoffcontainer zu schaffen, dass sie zusammen mit den entsprechenden Kunststoffcontainern eine Systemlösung ausbilden.

Die Aufgabe der Erfindung wird durch einen Kunststoffcontainer nach Anspruch 1 gelöst. Weiterhin wird die Aufgabe der Erfindung durch ein Containersystem mit den entsprechenden Unterstützungselementen gemäss Anspruch 5 gelöst.

Dabei haben die Massnahmen der Erfindung zunächst einmal zur Folge, dass beim Befüllen und Durchmischen des Kunststoffcontainers unerwünschte Strömungen vermieden werden. Weiterhin haben die Massnahmen der Erfindung zur Folge, dass sich nicht so leicht Sedimente ablagern und auch Totvolumina ausbilden können.

Vorteilhaft ist es, wenn nicht nur der Zylinderboden (Containerboden) angeschrägt ausläuft, sondern auch der Deckel, gemäss Anspruch 2. Vorteilhafterweise wird der Kunststoffcontainer gemäss Anspruch 3 durch Zusammenschweissen einer Kunststofffolie oder eines Kunststofffilms aus mehrschichtigem Kunststoff, vorzugsweise aus Polyethylen oder PTFE, vorzugsweise mit einer Dicke von 0.2mm bis 0.5mm inklusive Verschweissen der vier Folienecken hergestellt. Dabei ist es von Vorteil, wenn der Kunststoffcontainer gemäss Anspruch 4 eingeschweisste Kunststoff-Schlauchanschlüsse sowie eingeschweisste Schraubanschlüsse zur Befestigung von Mischern, optischen und/oder chemischen Sensoren wie zur Entleerung, Befüllung, Probeentnahme und/oder für ein Zusammenwirken mit anderer Ausrüstung aufweist.

Für das Containersystem ist es - gemäss Anspruch 6 - vorteilhaft wenn das Bodenunterstützungselement aus Chromstahl oder Kunststoff hergestellt ist und aus einem formschlüssigen Mantelring und einem abgewinkelten Auflageblech mit einem oder mehreren Durchgangslöchern besteht und mit einem Bodenblech mit einem oder mehreren Durchgangslöchern zusammengeschweisst oder auf eine andere Weise zusammengesetzt ist sowie wenn - gemäss Anspruch 7 - auf den abgewinkelten Aussenseiten des Bodenunterstützungselements ein oder mehrere Befestigungsbolzen angebracht sind.

Für die Unterstützungsvorrichtung ist es von Vorteil, wenn sie einen fahrbaren Tragrahmen umfasst und wenn beim Schliessen der Halbschalen die am Bodenunterstützungselement angebrachten Befestigungsbolzen mit entsprechend ausgenommenen Löchern an den Halbschalen verriegelt werden.

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können.

### Kurze Beschreibung der Zeichnungen

Weitere Einzelheiten, Vorteile und Merkmale des Gegenstandes der vorliegenden Erfindung ergeben sich aus der nachfolgenden Beschreibung der dazu gehörenden Zeichnungen, in denen - beispielhaft - erfindungsgemässe Messvorrichtungen erläutert werden. In den Zeichnungen zeigen:
- Figur 1: eine Darstellung eines Containers für biotechnische Reaktionen und/oder die Anzucht von Mikroorganismen und Zellkulturen gemäss einem bevorzugten Ausführungsbeispiel der vorliegenden Erfindung;
- Figur 2: einen Folienzuschnitt für einen Container nach Figur 1;
- Figur 3: eine Darstellung einer Bodenunterstützungselements für den Container nach Figur 1;
- Figur 4: eine Darstellung eines seitlichen Unterstützungselements für einen auf dem Bodenunterstützungselement gemäss Figur 3 angeordneten Container nach Figur 1 mit der Darstellung des Containers;
- Figur 5: eine dreidimensionale, schematische Darstellung des seitlichen Unterstützungselements gemäss Figur 4 in geschlossenem Zustand; und
- Figur 6: eine dreidimensionale, schematische Darstellung des seitlichen Unterstützungselements gemäss Figur 4 in offenem Zustand.

### Wege zur Ausführung der Erfindung

In Figur als Ganzes mit 10 bezeichnet ist ein Kunststoffcontainer mit einem Volumen von 50 bis 2000 Litern. Der Container 10 ist so geformt, dass er eine nahezu zylindrische faltenfreie Form erhält. Der integrierte Containerboden 12 und der Deckel 14 sind so geformt, dass sie 15° bis 70°, vorzugsweise 30° bis 40°, im Ausführungsbeispiel 35° bis 40° angeschrägt auslaufen und somit strömungsbrechend wirken, wenn der Kunststoffcontainer 10 mit Flüssigkeit gefüllt wird und/oder die Flüssigkeiten gemischt werden.

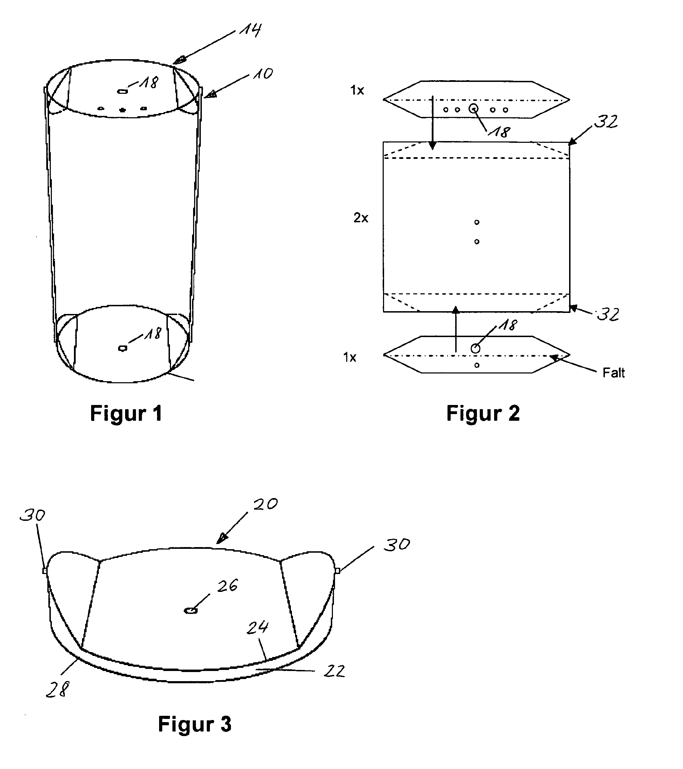

Im Folgenden werden nun Angaben über die Herstellung eines vorstehend beschriebenen Containers gemacht:
Der Kunststoffcontainer 10 ist so geformt und zugeschnitten, dass beim Zusammenschweissen eine nahezu zylindrische faltenfreie Form entsteht. Dies ist auch bedingt durch das Zusammenschweissen der vier Folienecken 32, wie in Figur 2 dargestellt. Die durch das Schneidemuster entstehenden sich verengenden, angeschrägten Enden dienen dem im Kunststoffcontainer 10 befindlichen Prozess als gewollte Flüssigkeits-Strömungsbrecher sowie Sedimentationshemmer und verhindern Totvolumina.

Auf den Enden, also Deckel 12 und Boden 14 sind Kunststoff-Schlauchanschlüsse 16 sowie Schraubanschlüsse 18 eingeschweisst, diese dienen zur Befestigung von Mischern 30, optischen und/oder chemischen Sensoren wie zur Entleerung, Befüllung, Probeentnahme und für ein Zusammenwirken mit anderer Ausrüstung.

Die Anschlüsse sind aus Kunststoff, im Ausführungsbeispiel aus Polyethylen (PE). Der Kunststofffilm, aus dem der Kunststoffcontainer 10 gefertigt ist, besteht im Ausführungsbeispiel aus mehrschichtigem Polyethylen oder PTFE, vorzugsweise mit einer Dicke von 0.2mm bis 0.5mm. Der Schraubanschluss 18 auf dem Zylinderdeckel 12 weist einen Innendurchmesser von 5mm bis 40mm auf, während die Aussendurchmesser der Schlauchanschlüsse 16 zwischen 4mm bis 25mm variieren.

In Figur 3 ist das Bodenunterstützungselement 20 für den vorstehend beschriebenen Kunststoffcontainer 10 dargestellt. Das Bodenunterstützungselement 20 für den Kunststoffcontainer 10 ist so geformt, dass es sich nahezu formschlüssig dem Zylinderboden 14 anpasst. Das Bodenunterstützungselement 20 ist vorzugsweise aus Chromstahl oder Kunststoff und besteht aus einem formschlüssigen Mantelring 22 und einem abgewinkelten Auflageblech 24 mit einem oder mehreren Durchgangslöchern 26 und ist mit einem Bodenblech 28 mit einem oder mehreren Durchgangslöchern nahtlos zusammengeschweisst.

Auf den abgewinkelten Aussenseiten des Bodenunterstützungselements 20 sind zwei Befestigungsbolzen 30 angebracht. Diese dienen zur Positionierung des Kunststoffcontainers 10 und auch als Befestigungs- und Montagehilfe.

Zum Containersystem für biotechnische Reaktionen und/oder die Anzucht von Mikroorganismen und Zellkulturen gehört vorteilhafterweise bei dieser Ausführungsform eine schliessbare, halbschalenförmige Unterstützungsvorrichtung 50 (Containersupport), die ein schnellstmögliches Installieren des Kunststoffcontainers 10 ohne Faltenbildung ermöglicht. Dies schafft für den Prozess optimale Voraussetzungen.

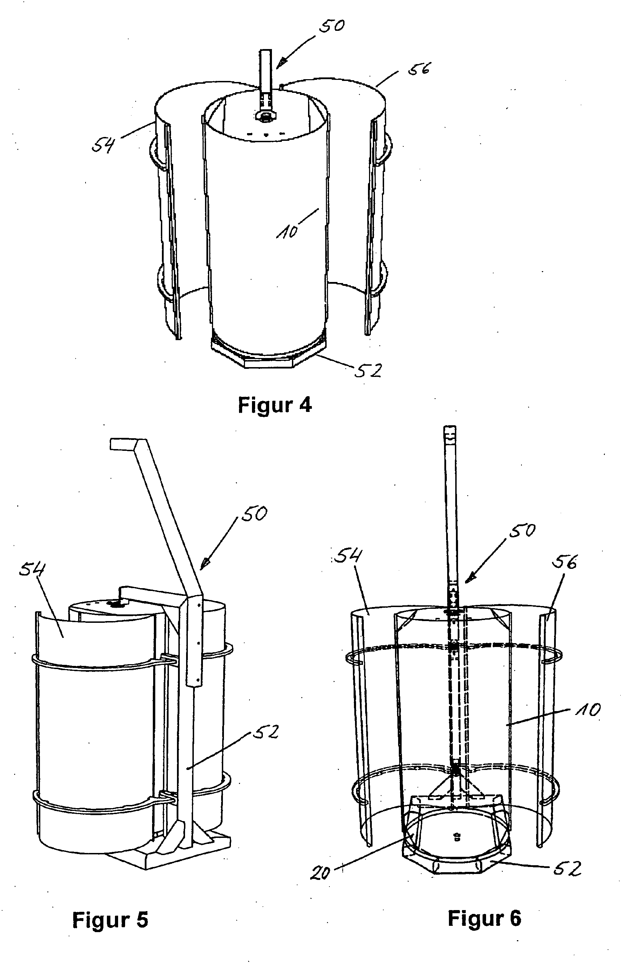

Die Unterstützungsvorrichtung 50 für den Kunststoffcontainer 10 mit einem fahrbaren Tragrahmen 52 besteht - wie in den Figuren 4 bis 6 dargestellt - aus zwei runden Halbschalen 54 und 56, die sich je nach Grösse des Kunststoffcontainers formschlüssig um den Kunststoffcontainer 10 sowie dem Bodenunterstützungselement 20 schliessen lassen. Beim Schliessen der Halbschalen 54, 56 werden zudem die am Bodenunterstützungselement 20 angebrachten Bolzen 30 mit entsprechend ausgenommenen Löchern an den Halbschalen 54 und 56 verriegelt und dienen als Montagehilfe und zur schnellen Positionierung des Kunststoffcontainers 10 für den Prozess von biotechnische Reaktionen und/oder der Anzucht von Mikroorganismen und Zellkulturen.

### Bezugszeichenliste

- 10: Kunststoffcontainer
- 12: Containerboden
- 14: Deckel
- 16: Kunststoff-Schlauchanschlüsse
- 18: Schraubanschlüsse
- 20: Bodenunterstützungselement
- 22: Mantelring
- 24: Auflageblech
- 26: Durchgangslöcher
- 28: Bodenblech
- 30: Befestigungsbolzen
- 32: Folienecken
- 38: Mischer
- 50: Unterstützungsvorrichtung
- 52: Tragrahmen
- 54: Halbschale
- 56: Halbschale

## Patentansprüche

1. Kunststoffcontainer (10) für biotechnische Reaktionen und/oder der Anzucht von Mikroorganismen und Zellkulturen, wobei der Kunststoffcontainer (10) eine zylindrische oder nahezu zylindrische Form aufweist, einen integrierten Containerboden (12) und einen Deckel (14) umfasst, wobei zumindest der Containerboden (12) so geformt ist, dass, er 15° bis 70°, vorzugsweise 30° bis 40°, angeschrägt ausläuft.

2. Kunststoffcontainer (10) nach Anspruch 1, **dadurch gekennzeichnet, dass** auch der Deckel (14) so geformt ist, dass, er 15° bis 70°, vorzugsweise 30° bis 40°, angeschrägt ausläuft.

3. Kunststoffcontainer nach Anspruch 2, **dadurch gekennzeichnet, dass** er durch Zusammenschweissen einer Kunststofffolie oder eines Kunststofffilms aus mehrschichtigem Kunststoff, vorzugsweise aus Polyethylen oder PTFE, vorzugsweise mit einer Dicke von 0.2mm bis 0.5mm an vier Folienecken hergestellt ist.

4. Kunststoffcontainer nach einem der Ansprüche 1 bis 3, **gekennzeichnet durch** eingeschweisste Kunststoff-Schlauchanschlüsse (16) sowie eingeschweisste Schraubanschlüsse (18) zur Befestigung von Mischern (30), optischen und/oder chemischen Sensoren wie zur Entleerung, Befüllung, Probeentnahme und/oder für ein Zusammenwirken mit anderer Ausrüstung.

5. Containersystem für biotechnische Reaktionen und/oder der Anzucht von Mikroorganismen und Zellkulturen, mit einem Kunststoffcontainer nach einem der Ansprüche 1 bis 4, mit einem Bodenunterstützungselement (20) das so geformt ist, dass es sich formschlüssig oder nahezu formschlüssig dem Containerboden (14) anpasst und mit einer schliessbaren, halbschalenförmigen Unterstützungsvorrichtung (50), wobei die zwei runden Halbschalen (54, 56) sich formschlüssig um den Kunststoffcontainer (10) sowie dem Bodenunterstützungselement (20) schliessen lassen.

6. Containersystem nach Anspruch 5, **dadurch gekennzeichnet, dass** das Bodenunterstützungselement (20) aus Chromstahl oder Kunststoff hergestellt ist und aus einem formschlüssigen Mantelring (22) und einem abgewinkelten Auflageblech (24) mit einem oder mehreren Durchgangslöchern (26) besteht und mit einem Bodenblech (28) mit einem oder mehreren Durchgangslöchern zusammengeschweisst oder auf eine andere Weise zusammengesetzt ist.

7. Containersystem nach Anspruch 6, **dadurch gekennzeichnet, dass** auf den abgewinkelten Aussenseiten des Bodenunterstützungselements (20) ein oder mehrere Befestigungsbolzen (30) angebracht sind

8. Containersystem nach einem der Ansprüche 5 bis 7, **dadurch gekennzeichnet, dass** die Unterstützungsvorrichtung (50) für den Kunststoffcontainer (10) einen fahrbarem Tragrahmen (52) umfasst.

9. Containersystem nach einem der Ansprüche 7 bis 8, **dadurch gekennzeichnet, dass** beim Schliessen der Halbschalen (54, 56) die am Bodenunterstützungselement (20) angebrachten Befestigungsbolzen (30) mit entsprechend ausgenommenen Löchern an den Halbschalen (54, 56) verriegelt werden.
